# EUROPÄISCHE PATENTSCHRIFT

(11) **EP 1 402 502 B1**
(45) Veröffentlichungstag und Bekanntmachung des Hinweises auf die Patenterteilung: **02.01.2008**
(21) Anmeldenummer: 02748605.9
(22) Anmeldetag: 25.06.2002
(51) Int. Cl.: G09B 23/28

(54) **GEBURTENSIMULATOR**
BIRTH SIMULATOR
SIMULATEUR D'ACCOUCHEMENT

(30) Priorität: 25.06.2001 DE 10130485; 23.01.2002 DE 10202504; 23.01.2002 DE 10202503; 23.01.2002 DE 10202502
(43) Veröffentlichungstag der Anmeldung: 31.03.2004
(73) Patentinhaber: Riener, Robert, 85591 Vaterstetten (DE); Burgkart, Rainer, 80469 München (DE)
(72) Erfinder: Riener, Robert, 85591 Vaterstetten (DE); Burgkart, Rainer, 80469 München (DE)
(74) Vertreter: Müller, Thomas
(86) Internationale Anmeldenummer: PCT/DE2002/002338
(87) Internationale Veröffentlichungsnummer: WO 2003/001482

(56) Entgegenhaltungen:
- WO-A-02/01536
- WO-A-02/29765
- US-A- 451 675
- US-A- 3 824 709
- US-A- 4 411 629
- US-A- 4 907 973
- US-B1- 6 238 215

## Beschreibung

Die Erfindung betrifft einen Geburtensimulator zum Nachbilden von vorgeburtlichen Behandlungsmethoden und zur Simulation ausgewählter Situationen beim Geburtsvorgang.

Die Ausbildung von Hebammen und Gynäkologen ist sehr aufwendig, da zu übenden Handgriffe oder die Anwendung von medizinischen Instrumenten (z. B. Saugglocke) aus verschiedenen Gründen nur sehr eingeschränkt an der Schwangeren selbst vorgenommen werden kann. Gerade in komplexen Notfallsituationen ist es nicht möglich oder ethisch vertretbar, unerfahrene Personen in die Geburtshilfe aktiv einzubeziehen. Weiterhin treten die unterschiedlichsten Problemfälle oft nicht vorhersehbar auf So müssen Hebammen und Gynäkologen über lange Zeiträume relativ passiv bei Geburten anwesend sein. Erst wenn die passive Ausbildung sehr weit fortgeschritten ist, kann mit der aktiven Ausbildung begonnen werden. Dabei müssen alle Handlungen von erfahrenem medizinischen Fachpersonal überwacht werden, um das Restrisiko für Mutter und Kind gering zu halten.

Um die gynäkologische Ausbildung zu unterstützen, werden bisher körperliche Modelle, Filme und Computeranimationen verwendet. Es gibt zusammensetzbare hartplastische Modelle, die eine räumliche Veranschaulichung anatomischer, physiologischer oder pathologischer Zusammenhänge ermöglichen. Es sind weiterhin weichelastische Modelle bekannt, die menschliche haptische Eigenschaften möglichst gut nachbilden sollen, d. h., in einem anatomiegerechten Mutterleib ist eine deformierbare Kinderpuppe angeordnet.

Die auszubildenden Hebammen und Gynäkologen können somit diese Modelle anfassen und bestimmte Grundhandgriffe üben und sich die räumlichen Zusammenhänge, wie z. B. Kindslagen, einprägen.

Da die aus dem Stand der Technik bekannten körperlichen Modelle, Filme oder Computeranimationen jedoch nur unzureichend für die realitätsnahe Ausbildung geeignet sind, besteht die Aufgabe der Erfindung in der Schaffung einer Vorrichtung zum Nachbilden von vorgeburtlichen Behandlungsmethoden und zur Simulation ausgewählter Situationen beim Geburtsvorgang, damit die erforderlichen Handgriffe wesentlich effektiver erlernt oder trainiert werden können. Diese Vorrichtung soll nachfolgend nur noch als Geburtensimulator bezeichnet werden.

Diese Aufgabe wird mit einem Geburtensimulator nach Anspruch 1 gelöst.

Ein Mutterleibstorso aus vorzugsweise weichelastischem Kunststoff oder einem Material mit vergleichbaren Eigenschaften, der die Form und Härte und somit Haptik eines natürlichen menschlichen Körpers aufweist, hat eine Gebärmutterhöhlung aus einem gummielastischen Material. Die Gebärmutterhöhlung ist so ausgebildet, daß in ihr ein Kindmodell aus weichelastischem Kunststoff einbringbar ist, wobei die Gebärmutterhöhlung und das Kindmodell in Form, Größe und Lage natürlichen Verhältnissen entsprechen. Erfindungsgemäß ist das Kindmodell mit einem motorischen Antrieb über eine Koppelvorrichtung mechanisch gekoppelt. Weiterhin ist eine programmierbare Ansteuervorrichtung zum Ansteuern des mechanischen Antriebs vorgesehen, so daß das Kindmodell nach einem frei programmierbaren Bewegungsablauf in der Gebärmutterhöhlung bewegbar oder durch den Geburtskanal aus dem Mutterleibstorso ausbringbar ist.

Zusätzlich sind eine Sensoranordnung zum Detektieren von Kräften und Bewegungen und ein Simulationsprogramm zur Simulation von Kraft- und Bewegungsfeedback vorgesehen. Wenn z. B. eine untersuchende Person mit den Händen auf den Mutterleibstorso drückt oder das Kindmodell direkt mit den Händen anfaßt oder mit einem medizinischen Instrument greift, werden die dabei eingeleiteten Kräfte von den Sensoren der Sensoranordnung gemessen. Dem Fachmann ist klar, daß gleichzeitig auch die Richtungen dieser Kräfte direkt oder indirekt bestimmbar sind. Die Sensoren sind an der Koppelvorrichtung und/oder an den Antriebselementen angeordnet. Auf dem Rechner der Steuervorrichtung ist das Simulationsprogramm implementiert, welches veranlaßt, daß die von der Sensoranordnung bereitgestellten Meßsignale in Kraft- und Bewegungsfeedback-Signale umrechnet werden. Diese Kraft- und Bewegungsfeedback-Signale werden der Steuervorrichtung zugeführt, die die Antriebselemente so regelt, daß das Kindmodell bei einer charakteristischen Krafteinwirkung durch eine Person mittels der Antriebselemente so bewegt wird, daß von dem Kindmodell eine adäquate Reaktionsbewegungen ausgeführt wird, die dem natürlichen Bewegungsverhalten des Kindes in der betreffenden medizinischen Situation entspricht. Der Hebamme oder der auszubildenden Person wird somit das Gefühl eines "lebendigen" Kindes vermittelt. Damit werden erstmals völlig neuartige Ausbildungsmethoden im Bereich der Schwangerenbetreuung und der Geburtshilfe ermöglicht. Lediglich durch eine Programmänderung, d. h. per Knopfdruck, können unterschiedlichste klinische Situationen und Bewegungs-Reaktionsmuster des natürlichen Kindes simuliert werden.

Der Vorteil der Erfindung besteht darin, daß eine Möglichkeit geschaffen wurde, unterschiedliche medizinische Situationen, d. h. zeitlich ablaufende Vorgänge vor und während der Geburt an einem anfaßbaren und sich bewegenden körperlichen Modell zu simulieren. Das Modell kann mit den Händen angefaßt werden, um z. B. zu empfinden, wie das Kind aus dem Geburtskanal gedrängt wird. Es ist ebenfalls möglich, den richtigen Einsatz von Instrumenten zu üben, wie z. B. die Verwendung einer Zange oder einer Saugglocke. Von besonderer Bedeutung ist die Möglichkeit, durch eine Programmänderung, d. h. "per Knopfdruck" eine andere medizinische Situation einstellen zu können.

Nach Anspruch 2 ist das Kindmodell mit mehreren ansteuerbaren mechanischen Antrieben gekoppelt, so daß auch kompliziertere Kindsbewegungen simulierbar sind, die den natürlichen Kindsbewegungen weitgehend entsprechen. Die verschiedenen Antriebe können auch vorzugsweise in einem Mehrgelenk-Roboter zusammengefaßt sein.

Nach Anspruch 3 ist zusätzlich eine optische Displayvorrichtung vorgesehen, die mit dem Rechner der Steuervorrichtung signaltechnisch verbundene ist. Diese Displayvorrichtung kann ein Monitor oder eine Datenbrille sein. Während einer Simulation können unterschiedliche visuelle Informationen eingespielt werden. Wenn z. B. eine spezielle Situation einer Geburt simuliert wird, läuft synchron ein Film ab, der z. B. die gleiche Situation bei einer natürlichen Geburt zeigt. Es können aber auch andere Darstellungsarten gewählt werden, wie z. B. eine Röngenfilmdarstellung.

Nach Anspruch 4 zeigt die optische Displayvorrichtung auch Hinweise und Zusatzinformationen an. Es ist für die Ausbildung besonders hilfreich, wenn z. B. zusätzliche Hinweise über gefährliche Situationen eingeblendet werden.

Nach Anspruch 5 ist zusätzlich ein Schallerzeuger zur Erzeugung von typischen Geräuschen vorgesehen. Es können sowohl Kindsgeräusche als auch Laute der Mutter eingespielt werden. Die Geräusche können synthetisch erzeugt werden oder auch natürlichen Ursprungs sein, d. h. es handelt sich um Tonbandaufnahmen, die während einer adäquaten natürlichen Situation aufgenommen wurden. Durch diese Maßnahme wird für die auszubildende Person ein sehr wirklichkeitsnaher Eindruck erzeugt, wenn z. B. bei einer heftigen Wehentätigkeit zeitgleich ein Stöhnen der Gebärenden eingespielt wird.

Nach Anspruch 6 sind die Schallerzeuger im Mutterleibstorso integriert. Damit können insbesondere die durch das Kind verursachten Geräusche sehr echt simuliert werden.

Nach Anspruch 7 wird ein Kindmodell bereitgestellt, das zur Verwendung in einem Geburtensimulator nach den Ansprüche 1 bis 6 bestimmt ist. Das Kindmodell weist im Halsbereich und/oder im Bereich des Schädeldaches, das aus verformbaren Segmenten besteht, Weg- und/oder Kraft- und/oder Drucksensoren auf, die signaltechnisch mit dem Rechner der Steuervorrichtung verbunden sind.

Dieses Kindmodell ermöglicht bei der Anwendung mit einem Geburtensimulator nach Anspruch 1 bis 6, daß zusätzliche Informationen gewonnen werden, die den Lerneffekt verbessern. So können z. B. die Krafteinleitungen am Kind aufgezeichnet werden, während eine ausgebildete Person am Geburtensimulator eine Geburt simuliert. Ebenso wird bei einer auszubildenden Person verfahren. Anschließend können die beiden Kraftverläufe verglichen und bewertet werden.

Eine bevorzugte Stelle für die Applikation eines Kraft- und/oder Momentensensors ist der Hals des Kindmodells. Eine bevorzugte Stelle zur Applikation von Wegaufnehmern oder Drucksensoren ist der Schädelbereich des Kindmodells. Wenn Wegaufnehmern vorgesehen sind, ist der Schädel analog zu einem natürlichen Kindsschädel verformbar und weist verschiebbare Schädelsegmente auf, deren Verschiebungen durch die Wegaufnehmer gemessen werden.

Die Sensorinformationen des Kindmodells werden dazu verwendet, mehr und genauere Kraft- und/oder Momenteninformationen zur Berechnung der Reaktionskräfte und der dazugehörigen Reaktionsbewegungen bereitzustellen. Es soll weiterhin erwähnt werden, daß der Fachmann bei Bedarf auch weitere Sensoren an geeigneten Stellen am Kind oder auch am Mutterleibstorso anordnen wird, wenn es zur Signalgewinnung bei der Umsetzung einer konkreten Bewegungssimulation erforderlich ist. So können z. B. auf dem Bauchbereich des Mutterleibstorsos Drucksensoren angeordnet werden.

Die Erfindung wird nachfolgend an Hand von Ausführungsbeispielen in Verbindung mit schematischen Zeichnungen näher erläutert.
- Fig. 1: zeigt eine schematische Darstellung einer ersten aktive Ausführungsform der Erfindung.
- Fig. 2: zeigt eine schematische Darstellung einer zweiten aktive Ausführungsform der Erfindung.
- Fig. 3: zeigt eine schematische Darstellung einer dritten aktive Ausführungsform der Erfindung.
- Fig. 4: zeigt eine schematische Darstellung einer vierten aktive Ausführungsform der Erfindung.
- Fig. 5: zeigt eine schematische Darstellung einer fünften aktive Ausführungsform der Erfindung.
- Fig. 6: zeigt eine schematische Darstellung einer sechsten aktive Ausführungsform der Erfindung.
- Fig. 7: zeigt eine schematische Darstellung einer erste Ausführungsform einer zweiten Erfindung.
- Fig. 8: zeigt eine schematische Darstellung einer zweiten Ausführungsform einer zweiten Erfindung.
- Fig. 9: zeigt eine interaktive Ausführungsform der Erfindung.
- Fig. 10: zeigt einen ersten Regelalgorithmus für eine interaktive Ausführungsform der Erfindung.
- Fig. 11: zeigt einen zweiten Regelalgorithmus für eine interaktive Ausführungsform der Erfindung.

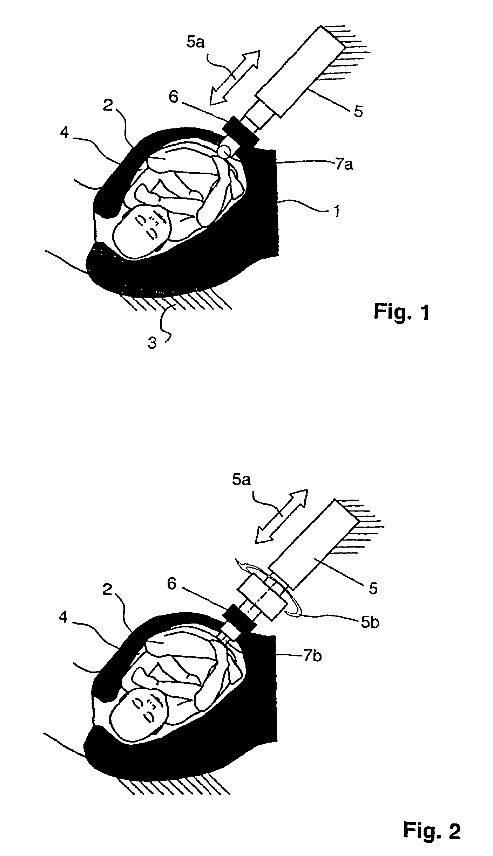

Die Fig. 1 zeigt den Querschnitt eines Geburtensimulators in Form und Größe eines Mutterleibstorsos 1 einer Schwangeren mit einem Kindmodell 2. Der Mutterleibstorso 1 ist in einem Bereich 3 auf einer Unterlage, z. B. ein Tisch, fest angeordnet. Das Kindmodell 2 befindet sich in einer Höhlung 4, welche die Gebärmutter simuliert. Der Mutterleibstorso 1 und das Kindmodell 2 sind aus einem weichelastischen Kunststoff ausgebildet. An dem Kindmodell 2 ist ein Linearantrieb 5 über einen Kraftsensor 6 angekoppelt. Der Linearantrieb 5 hat eine interne Positionserkennungsvorrichtung in Form eines Längenmeßsystems und ermöglicht eine wechselseitige Bewegung in Pfeilrichtung 5a. Der Kraftsensor 6 ist über ein Kugelgelenk 7a mit dem Kindmodell 2 verbunden. Wenn der Linearantrieb 5 das Kindmodell 2 aus dem Geburtskanal drängt, muß eine auszubildende Person das Kindmodell 2 wie bei einer richtigen Geburt halten und fuhren. Die dabei wirkenden Kräfte werden von dem Kraftsensor 6 erfaßt und als Kraftmeßsignale von einer Auswerteelektronik verarbeitet und als Kraftmeßdaten in einer Speichervorrichtung gespeichert. Die bei einer simulierten Geburt verfolgbaren Kraft- und Bewegungsverläufe werden mit gespeicherten Norm-Kraft- und -Bewegungsverläufen verglichen. Aus den Abweichungen zwischen den verfolgten Kraft- und Bewegungsverläufen und den gespeicherten Norm-Kraft- und - Bewegungsverläufen können Rückschlüsse auf den Trainingserfolg der auszubildenden Person gezogen werden.

Die Fig. 2 zeigt eine prinzipiell gleiche Anordnung wie Fig. 1, wobei der Linearantrieb 5 neben der wechselseitigen Bewegung 5a zusätzlich noch eine Drehbewegung 5b ermöglicht. Im Unterschied zu Fig. 1 ist die Koppelstelle zwischen dem Kindmodell 2 und dem Kraftsensor 6 als ein torsionssteifes, federelastisches Glied 7b ausgebildet.

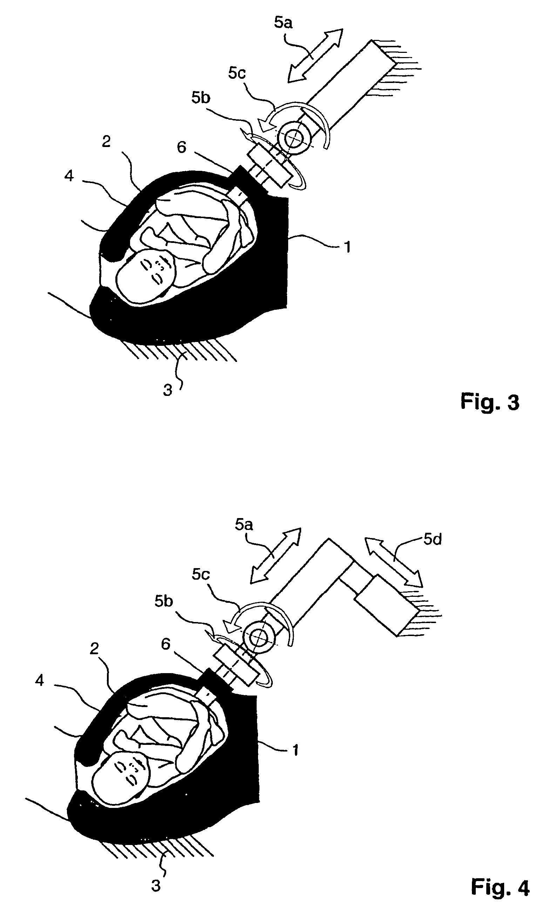

Die Fig. 3 zeigt die prinzipiell gleiche Anordnung wie Fig. 2, wobei der Linearantrieb 5 neben den Bewegungen 5a und 5b noch eine zusätzliche Schwenkbewegung 5c ermöglicht. Im Unterschied zu Fig. 1 oder 2 ist die Koppelstelle zwischen dem Kindmodell 2 und dem Kraftsensor 6 starr ausgebildet.

Die Fig. 4 zeigt die prinzipiell gleiche Anordnung wie Fig. 3, wobei der Linearantrieb 5 neben den Bewegungen 5a, 5b und 5c noch eine zusätzliche Bewegung 5d ausführt, die rechtwinklig zur Richtung der Bewegung 5a ist. Die Koppelstelle zwischen dem Kindmodell 2 und dem Kraftsensor 6 ist ebenfalls starr ausgebildet.

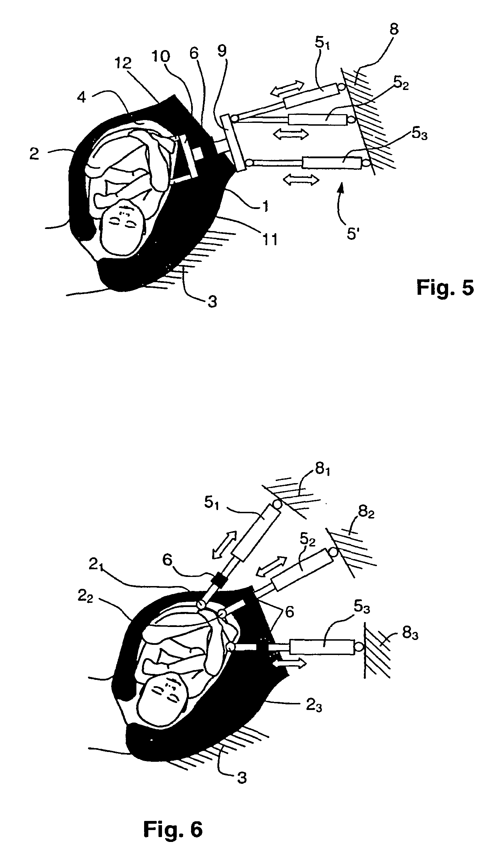

Die Fig. 5 zeigt einen Linearantrieb 5, der aus drei Einzelantrieben 5₁, 5₂ und 5₃ besteht, deren Endabschnitte schwenkbar an einem Widerlager 8 und an einer Platte 9 angelenkt sind. Die Platte 9 ist über den Kraftsensor 6 mit einer Zweipunkthalterung 10 starr verbunden. Die Zweipunkthalterung 10 fixiert das Kindmodell 2 an den Punkten 11 und 12.

Die Fig. 6 zeigt einen Linearantrieb 5, der, wie unter Bezugnahme auf Fig. 5 beschrieben, ebenfalls aus drei Einzelantrieben 5₁, 5₂ und 5₃ besteht, deren Endabschnitte an je einem Widerlager 8₁, 8₂ und 8₃ verschwenkbar angelenkt sind. Die sich in Richtung des Kindmodells 2 erstreckenden Endabschnitte sind an diesem an verschiedenen Punkten 2₁, 2₂ und 2₃ verschwenkbar angelenkt.

Dem Fachmann ist an Hand der schematischen Zeichnungen und der vorstehenden Erläuterungen klar, wie mittels der verschiedenen Antriebe unterschiedlicher Freiheitsgrade realitätsnahe Bewegungen des Kindmodells bewirkt werden können.

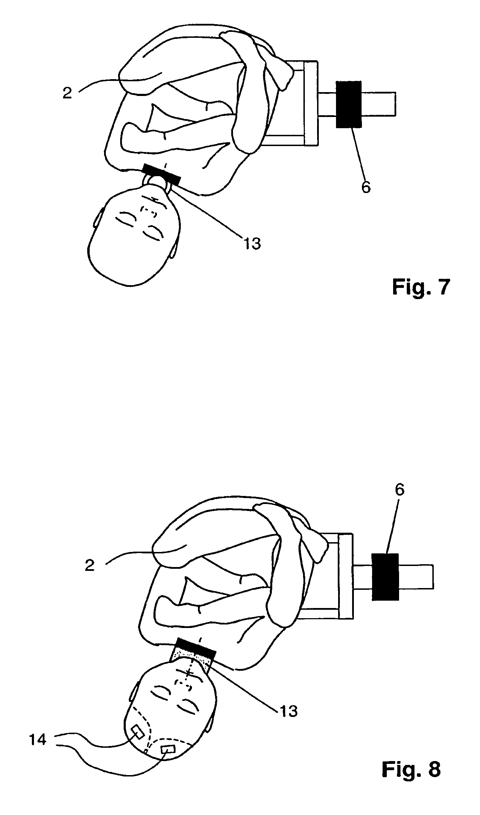

Die Fig. 7 zeigt ein Kindmodell 2, dessen Kopf über einen Kraft- und Drehmomentensensor 13 mit dem Rumpf verbunden ist. Bei der Geburtensimulation ist es besonders wichtig, Handgriffe am Kopf des Kindmodells 2 zu üben. Dabei wird der Hals des Kindes besonders beansprucht. Es ist daher bei der Überwachung einer simulierten Geburt von besonderer Bedeutung, die Koptbriffe zu kontrollieren, was mit dieser Ausführungsform eines Kindmodells möglich ist. Für die Übertragung der elektrischen Meßsignale stehen dem Fachmann drahtgebundene sowie drahtlose Übertragungsverfahren zur Verfügung.

Die Fig. 8 zeigt ein Kindmodell 2, an dessen Kopf gegenüber der Darstellung in Fig. 7 zusätzlich Weg- und Drucksensoren 14 im Bereich der Schädelknochen angeordnet sind. Weiterhin sind einzelne Abschnitte der Schädeldecke des Kindmodells 2 verschiebbar ausgebildet. Wird z. B. eine Geburt simuliert, verschieben sich die Abschnitte der Schädeldecke wie beim natürlichen Geburtsprozeß, wobei beim Training zu beachten ist, daß die Verschiebungen medizinisch vorgegebene Grenzen nicht überschreiten. Mittels der Weg- und Drucksensoren sind während einer simulierten Geburt die Verschiebungen und Drücke erfaßbar, und aus den gewonnenen Daten kann beurteilt werden, ob die auszubildende Person den Kopf des Kindmodells richtig hält und führt. Dem Fachmann ist es freigestellt, geeignete Weg- und Drucksensoren und die dafür geeignete Signalverarbeitungstechnik auszuwählen und einzusetzen.

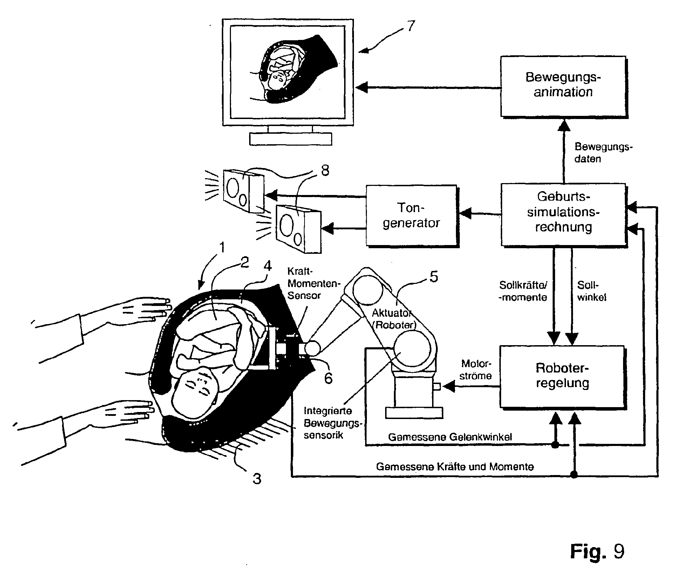

Die Fig. 9 zeigt schematisch den Querschnitt eines Geburtensimulators in Form eines Bauchtorsos 1 einer Schwangeren mit einem Kindmodell 2. Der Bauchtorso 1 ist in einem Bereich 3 auf einer Unterlage, z. B. ein Tisch, fest angeordnet. Das Kindmodell 2 befindet sich in einer Höhlung 4, welche die Gebärmutter simuliert. Der Bauchtorso 1 und das Kindmodell 2 sind aus einem weichelastischen Kunststoff ausgebildet. An dem Kindmodell 2 ist ein sechsgelenkiger Roboter 5 mit serieller Kinematik über einen Sechskomponenten-Kraft-Momenten-Sensor 6 angekoppelt.

Wenn der Roboter 5 das Kindmodell 2 bei einer simulierten Geburt aus dem Geburtskanal drängt, muß eine auszubildende Person das Kindmodell 2 wie bei einer realen Geburt erfassen und führen. Die dabei eingeleiteten Kräfte und Momente werden von dem Sechskomponenten-Kraft-Momenten-Sensor 6 erfaßt, in elektrische Signale umgewandett und einer Steuer- und Regelvorrichtung zugeführt.

Der Roboter weist eine serielle Kinematik auf, d. h., mehrere Robotersegmente sind über aktiv angetriebene Drehachsen miteinander seriell verbunden. Die Richtungen der Drehachsen sind so gewählt, daß der Endeffektor des Roboters mit dem an den Sechskomponenten-Kraft-Momenten-Sensor 6 angekoppelten Kindmodell in sechs Freiheitsgraden (drei Positionen und drei Orientierungen) bewegt werden kann. Die Winkelstellungen des Roboters werden über interne Gelenkwinkelsensoren erfaßt und der Steuer- und Regelvorrichtung zugeführt. Aus den Gelenkwinkeldaten wird die Lage des Kindmodells 2 im Raum bestimmt.

Wenn eine Person das Kindmodell 2 mit den Händen indirekt über die elastische Baudekke 6 oder auch direkt berührt, wird von der Person durch die von Hand oder mittels eines medizinischen Instrumentes auf das Kindmodell 2 aufgebrachten Kräfte eine Bewegung induziert. Das Kindmodell 2 reagiert darauf nach einem Bewegungsmuster, welches einer realen Reaktionsbewegung eines natürlichen Kindes entspricht.

Die Bewegungsinduktion des Kindmodells kann prinzipiell nach zwei Methoden erfolgen:

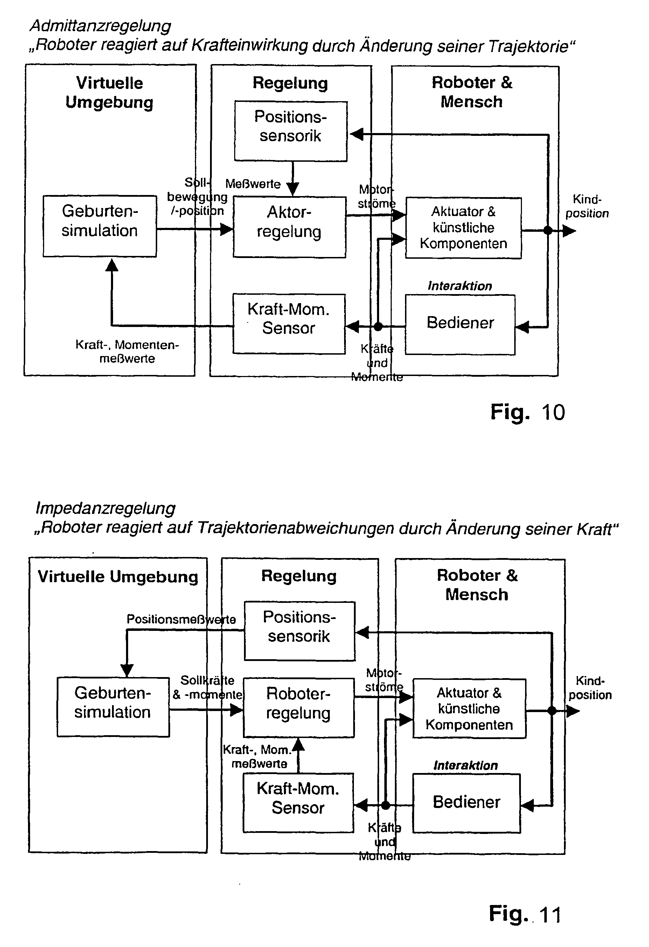

Die erste Methode ist die in Fig. 10 dargestellte Admittanzregelung, nach der die bei Berührung des Kindmodells 2 eingeleiteten Kräfte durch den Sechskomponenten-Kraft-Momenten-Sensor 6 erfaßt und zu dem Rechner mit dem Simulationsprogramm übertragen werden. Dort wird die resultierende Bewegung des Kindmodells 2 berechnet und als Sollgröße an die Roboterregelung weitergegeben. Die Roboterregelung vergleicht die errechnete Sollbewegung mit der gemessenen Roboterbewegung und speist den Roboter mit Motorströmen so, daß der Fehler zwischen gemessener und errechneter Bewegung minimal wird.

Die zweite Methode ist die in Fig. 11 dargestellte Impedanzregelung, nach der die durch die Wirkung von Kräften und Momenten erzwungenen Positions- und Orientierungsänderungen erfaßt und zu dem Rechner mit dem Simulationsprogramm übertragen werden. Daraufhin werden die korrespondierenden Kräfte und Momente berechnet und als Sollgrößen an die Roboterregelung weitergeleitet. Die Roboterregelung vergleicht die berechneten Sollkräfte und -momente mit den tatsächlich auftretenden Kräften und Momenten und bewegt den Roboter so, daß der auftretende Fehler minimal wird.

Das Simulationsprogramm für die Geburtssimulationsberechnung beinhaltet somit ein Computermodell, welches die biomechanischen Beziehungen zwischen Becken, Gebärmutter, Bändern, Sehnen, Haut und Muskulatur der Mutter und dem Körper des Kindmodells enthält. Es beschreibt die statischen und dynamischen Zusammenhänge zwischen den auftretenden Kräften und Momenten, die eine Person, wie z. B. die auszubildende Hebamme, auf das Kindmodell aufbringt, und die Lagen und Bewegungen des Kindes hinsichtlich den Raumpositionen/-orientierungen und deren Ableitungen, d. h. Geschwindigkeiten und Beschleunigungen relativ zum Körper der Mutter. Dadurch können entweder aus den gemessenen Kräften und Momenten die resultierenden Bewegungen des Kindmodells 3 (Admittanzregelung) oder aus der gemessenen Kindsbewegung die dazugehörigen Kräfte und Momente (Impedanzregelung), die auf die agierende Person übertragen werden, berechnet werden.

Durch die Regelung der mechanischen Kraftaktorik 5 hat die agierende Person den haptisch subjektiven Eindruck einer realen Reaktion. Durch entsprechende Parameterwahl in der Geburtssimulationsberechnung lassen sich nicht nur normale Geburtsvorgänge oder Kindsbewegungen simulieren, sondern auch seltene Situationen und Problemfälle darstellen und anschaulich vermitteln.

Bei der Ausführungsform der Erfindung nach Fig. 9 werden weiterhin aus den in der Geburtensimulation aufbereiteten Bewegungsinformationen in einer Bewegungsanimationsrechnung die Bewegungen und Verformungen der anatomischen Komponenten, wie z. B. Becken, Gebärmutter, Bänder, Sehnen, Haut, Muskulatur der Mutter und des Kindes, ermittelt und in Echtzeit auf einem Monitor 7 visualisiert. Es können unterschiedliche Darstellungsarten gewählt werden, wie z. B. die gezeigte röntgenbildähnliche Darstellung oder eine ultraschallbildartige Darstellung, wobei z. B. besonders gefährdete Abschnitte oder Verletzungen farbig hervorgehoben werden können. Gleichfalls ist es möglich, zwischen verschiedenen Darstellungsarten umzuschalten. Da die visuellen Informationen zeitgleich mit den haptischen Informationen der agierenden Person übermittelt werden, entsteht für diese ein sehr realistischer Gesamteindruck.

Bei der Ausführungsform der Erfindung nach Fig. 9 werden zusätzlich aus den biomechanischen Berechnungen auch noch Schmerzgrenzwerte ermittelt, die bei Überschreitung einen Befehl zum Abspielen eines Soundsamples auslösen. Diese Soundsamples sind in einem Speicher abgelegt und werden nach Anforderung aufgerufen und über ein Stereolautsprechersystem 8 wiedergegeben. Es ist für die agierende Person von nachhaltiger lernpsychologischer Wirkung, wenn z. B. bei einem falschen Handgriff ein Schmerzenslaut ertönt.

Nachfolgend werden weitergehende Hinweise zur Realisierung des Aktuators und dessen Steuerung gegeben.

Das Aktuator wurde mittels eines sechsachsigen Stäubli Industrieroboters RX90 verifiziert. Der originale Steuerrechner des Roboters benötigt verhältnismäßig lange Zykluszeiten von mehr als 16 ms. Für einen stabilen Betrieb des Geburtensimulators und eine störungsfreie und wirklichkeitsnahe Darstellung biomechanischer Eigenschaften sind u. U. höhere Abtastraten im kHz-Bereich unter Echtzeitbedingungen und eine hohe Rechenleistung zur Implementierung der modellbasierten Regelungsverfahren notwendig. Aus diesem Grund wurde parallel eine PC basierte Ansteuerung aufgebaut. Über einen Umschalter können die Winkel-, Kraft- und Momentensensoren und die Analogsignale für die elektronischen Gelenkverstärker vom Stäubli-Rechner auf den PC umgeschaltet werden, wo eine Signalerfassung/-ausgabe durch entsprechende PCI-Karten erfolgt. Durch Konfiguration der elektronischen Gelenkverstärker auf eine Stromregelung kann so auf dem PC die vorstehend beschriebene, auf Gelenkmomentenschnittstellen beruhende Regelung implementiert werden. Aufgrund der wesentlich höheren Rechenleistung des PCs kann damit die Abtastzeit auf 250 µs verkürzt werden, was zu wesentlich besseren Ergebnissen als mit der Original-Architektur führt. Vorteilhaft bei diesem Vorgehen ist, daß alle anderen Original-Komponenten wie die Gelenkverstärker, die Sicherheitselektronik für Bremsen und den Nothaltkreis sowie die Stromversorgung erhalten bleiben und weiterhin verwendet werden können.

Nachfolgend werden weitergehende Hinweise zur Realisierung des biomechanische Modells gegeben.

Zur Umsetzung der Erfindung muß das zu Grunde liegende biomechanische Modell entwickelt werden. Im biomechanischen Modell wird der Zusammenhang zwischen den von außen (Bediener) auf das Kind eingeprägten Lasten, d.h. Kräfte und Momente (Ursache) und der zugrundeliegenden Bewegung oder Position (Wirkung) dargestellt. Ob nun bei der mathematischen Darstellung das Ursache-Wirkungs- oder Wirkungs-Ursache-Prinzip beschrieben wird, hängt dabei von der Art der Regelung ab. Bei der Admittanzregelung wird ein inverses Modell benötigt, d.h. es werden die Ursachen (Kräfte, Momente) als Funktion der Wirkungen (Positionen, Geschwindigkeiten) berechnet. Umgekehrt werden bei der Impedanzregelung in einem direkten dynamischen Modell (auch Vorwärtsmodell genannt) Wirkungen aus den Ursachen berechnet. Die Berechnungsweise, d.h. ob invers oder direkt, ist für die Modellierungsweise nur von untergeordneter Bedeutung. Im folgenden wird ein Modellierungsbeispiel in direkter Richtung skizziert:

Das Kind besitzt eine bestimmte anatomisch bedingte Form/Geometrie, die im einfachsten Fall als starr und steif betrachtet werden kann. Sinnvoll ist aber die Annahme, daß sich die Form des Kindes bei Kräften, die vom Bediener oder durch den Kontakt mit Abschnitten des Mutterleibs auf das Kind ausgeübt werden, passiv viskoelastisch verformt. Ebenso besitzt der Mutterleib, bestehend aus Gebärmutter, Bauch, Geburtskanal, Becken usw. bestimmte geometrische und viskoelastische Eigenschaften.

Wird nun vom Bediener eine Kraft und/oder ein Moment auf das Kind ausgeübt, so übertragen sich diese Lasten über das Kind an die Kontaktstellen Kind-Mutter (z.B. in der Gebärmutter oder im Geburtskanal). An diesen Stellen kommt es zu verformungs- und reibungsbedingten Relativbewegungen. Je nachdem wie nun die geometrischen und viskoelastischen Eigenschaften der entsprechenden (d.h. involvierten) kindlichen und mütterlichen Körperabschnitte gestaltet sind, kommt es zu mehr oder weniger deutlichen Bewegungen (deutlich heißt hier: schnell oder in merklichem Ausmaß). Die Position des Kindes kann also durch das Aufbringen von Lasten von außen (über Vagina oder Bauchdecke) beeinflußt werden. Dabei ist anzumerken, daß sich das Kind auch ohne Zutun von außen - allein durch die Kontraktion der Gebärmutter - aus dem Mutterleib bewegen kann, sofern keine größeren mechanischen oder muskulären Widerstände auftreten. Wenn jedoch eine Verengung des Geburtskanals vorliegt, oder der Schädel des Kindes durch eine ungünstige Schräglage im Geburtskanal "verkantet", so muß der Bediener die Kräfte und Momente so einleiten, daß er entweder die "austreibenden Kräfte" erhöht (z.B. durch Abwärtsdrücken auf der Bauchdecke der Mutter) oder die Verkantung durch Drehbewegungen des Kindskopfes (indem er von außen in den Geburtskanal greift) beseitigt.

Das biomechanische Modell muß nicht notwendigerweise alle anatomischen Komponenten und Formen explizit beinhalten. Es reicht eine gewisse "abstrahierte" Darstellung der mathematischen Zusammenhänge zwischen eingeprägten Kräften und resultierenden Bewegungen. D.h. eine mathematische Funktion beschreibt welche Position, Orientierung und Geschwindigkeit sich ergibt, wenn eine Kraft und ein Moment an einer bestimmten Stelle am Kind in eine bestimmte Richtung wirkt. Dabei ist die Multidimensionalität des Problems zu beachten. D.h. die eingeleiteten Kräfte und Momente wirken in 3D Richtungen und können an beliebiger Stelle der Oberfläche des Kindes angreifen. Die resultierenden Positionen, Orientierungen und Geschwindigkeiten sind ebenso in 3D anzugeben. Die Beziehung zwischen Kraft/Moment und Lage/Bewegung hängt zudem noch von der momentanen Position des Kindes in der Gebärmutter bzw. im Geburtskanal ab. Diese mathematischen Beziehungen können auf der Basis linearer oder nicht-linearer algebraischer Gleichungen leicht hingeschrieben werden. Schwierig ist jedoch die korrekte Parametrisierung. Die Wahl der Parameter bestimmt, wie realitätsnah der normale oder pathologische Geburtsvorgang simuliert werden kann. Die Parameter können auf der Basis theoretischer Überlegungen abgeschätzt oder experimentell/messtechnisch gewonnen werden.

Nachfolgend werden weitergehende Hinweise zur Realisierung des grafischen Displays gegeben. Mit einem Monitor werden interne anatomische Komponenten, wie Beckenknochen, Gebärmutter, Plazenta, Muttermund, Blutgefäße sowie das Kind visualisiert. Optional kann der Monitor zusammen mit einer Schutterbrille auch im Stereomodus betrieben werden. Die Bewegungsanimation erfolgt synchron mit den Bewegungen des Geburtensimulators. Der Bediener hat dadurch die Möglichkeit, die körperinternen, anatomischen und biomechanischen Zusammenhänge auch während der Bewegung des Kindes zu studieren. Die Visualisierung erfolgt auf der Basis segmentierter und 3D-rekonstruierter CT- und MRT-Aufnalunen. Die rekonstruierte anatomische Darstellung stellt eine Zusatzinformation dar, die bei der medizinischen Ausbildung einen hohen didaktisch Stellenwert besitzt, bei einer realen Geburt jedoch nicht ersichtlich gemacht werden kann. In der klinischen Routine werden in der Regel nur Ultraschalltechniken zur Beobachtung und Beurteilung der Geburt verwendet. Solche Ultraschallaufnahmen können auf der Basis von zusammengesetzten Einzelbildern, die synchron mit der Geburt ablaufen, in der Bewegungsanimation simuliert werden.

In der grafischen Animation werden bewegungssynchrone Lageänderungen der Körpersegmente, Verlaufsänderungen von Blutgefäßen oder der Nabelschnur, sowie Verformungen von Muskeln, Gebärmutter, Plazenta usw. berücksichtigt. Eine Visualisierung solcher Bewegungsvorgänge ist durch so genannte "kinematische CT und MRT Aufnahmen" möglich. Hierbei handelt es sich aber nur um eine cinematografische Technik, die keine interaktive Bedienung in mehr als einem Freiheitsgrad zuläßt und daher für eine Anwendung im VR-Bereich nur begrenzt geeignet ist (Dupuy et al. 1997; Witonski und Goraj 1999). Eine Alternative stellt eine modellbasierte Animation dar. Darin werden alle Komponenten in ihren relevanten geometrischen und viskoelastischen Eigenschaften und ihrem mechanischen Zusammenspiel modelliert. Für eine realitätsnahe Simulation sind aber FE-Rechnungen und komplexe Mehrkörper-Kontaktmodelle notwendig, die den simulationstechnischen Aufwand erheblich vergrößern und so die Echtzeitfähigkeit des Systems gefährden können.

Empfohlen wird daher ein kombiniertes Verfahren, bei dem Bilddaten ebenso wie anatomische Modellbetrachtungen zum Einsatz kommen. Der Ansatz besteht darin, Geometriedaten, die aus zahlreichen diskreten Geburtsmomenten rekonstruiert werden, so zu inter- und extrapolieren, daß jede beliebige Kindsposition in jedem wichtigen Freiheitsgrad dargestellt werden kann. Die Inter- und Extrapolationen können dabei modellunterstützt erfolgen, indem beispielsweise die Volumenerhaltung oder Längenkonstanz bestimmter Körperabschnitte berücksichtigt wird. Da dies mit verhältnismäßig geringem Rechenaufwand möglich ist, können echtzeitfähige und glatte Bewegungsabläufe in jeder beliebigen Richtung erzielt werden.

Nachfolgend werden weitergehende Hinweise zur Realisierung des akustischen Displays gegeben. Bei der Geburt treten eine Reihe verschiedener akustischer Signale auf, die von Lautsprechern erzeugt werden. Dazu zählen Schmerzschreie der Mutter, Geräusche beim Austritt des Kindes, akustische dargestellte Signale, wie z.B. Wehentätigkeit der Mutter und EKG des Kindes. Die Lautsprecher können in der Nähe der künstlichen Körperabschnitte platziert oder in die Körperabschnitte so eingebaut werden, daß sie von außen nicht sichtbar sind.

Die Geburtsgeräusche können an mehreren Probandinnen während der Geburt aufgenommen werden. Zur Darstellung der Geräusche müssen Modelle gefunden werden, die die Art des Geräusches mit der zugnmdeliegenden Situation und der ausgeführten Bewegungsaktionen des Bedieners in Zusammenhang bringen. Auf der Basis der Erfahrung zahlreicher Gynäkologen können diese Zusammenhänge zunächst mit Hilfe von linguistischen Variablen qualitativ beschrieben werden. Mit der Methode der Fuzzy-Logik können dann aus den linguistischen Angaben quantitative Zusammenhänge hergeleitet werden.

## Patentansprüche

1. Geburtensimulator mit nachfolgenden Merkmalen:
- einem Mutterleibstorso **(1)** aus flexiblem Material,
- einem Kindmodell **(2)** aus flexiblem Material, das in dem Mutterleibstorso **(1)** angeordnet ist, wobei vorzugsweise die natürlichen Form- und Größenverhältnisse und Haptik eingehalten werden, das Kindmodell **(2)** über eine
- Koppelvorrichtung **(7)** mit einem steuerbaren Antrieb **(5)** verbunden ist, um das Kindmodell **(2)** im Mutterleibstorso **(1)** zu bewegen oder durch den Geburtskanal aus dem Mutterleibstorso **(1)** zu drängen, und
- eine programmierbare, einen Rechner aufweisende Steuervorrichtung zum Steuern des Antriebs **(5)** vorgesehen ist,
**dadurch gekennzeichnet, dass**
- eine Sensoranordnung zum Detektieren von Kräften und Bewegungen vorgesehen ist, die durch eine untersuchende Person mit den Händen oder mit medizinischen Instrumenten auf den Mutterleibstorso **(1)** oder auf das Kindmodell **(2)** aufgebracht werden, wobei die Sensoren der Sensoranordnung an der Koppelvorrichtung **(7)** und/oder an den Antriebselementen **(5)** angeordnet sind und
- die Steuervorrichtung eine
- Admittanzregelung oder eine
- Impedanzregelung
zum Ansteuern der Antriebselemente **(5)** aufweist, wobei die Steuervorrichtung so ausgebildet ist, dass die von der Sensoranordnung bereitgestellten Messsignale dem Rechner zugeführt werden, in welchem
- ein Simulationsprogramm abgelegt ist, welches im Zusammenwirken mit der Admittanzregelung oder mit der Impedanzregelung der Steuervorrichtung veranlasst, dass das Kindmodell **(2)** bei äußerer Krafteinwirkung mittels der Antriebselemente **(8)** so bewegt wird, dass das Kindmodell **(2)** Bewegungen ausführt, die dem natürlichen Bewegungsverhalten eines Kindes im Mutterleib bei der betreffenden Untersuchung oder bei dem betreffenden Geburtsabschnitt entsprechen.

2. Geburtensimulator nach Anspruch 1, **dadurch gekennzeichnet, daß** zur Bewegung des Kindmodells **(2)** mehrere Koppelvorrichtungen **(7)** und mehrere steuerbare Antriebe **(5)** vorgesehen sind.

3. Geburtensimulator nach einem der vorhergehenden Ansprüche, **dadurch gekennzeichnet, daß** eine mit der Steuervorrichtung signaltechnisch verbundene optische Displayvorrichtung vorgesehen ist, die die Kindsbewegungen in Echtzeit zeigt.

4. Geburtensimulator nach Anspruch 3, **dadurch gekennzeichnet, daß** die optische Displayvorrichtung Hinweise und Zusatzinformationen anzeigt.

5. Geburtensimulator nach einem der vorhergehenden Ansprüche, **dadurch gekennzeichnet, daß** ein mit der Steuervorrichtung signaltechnisch verbundene Schallerzeuger zur Erzeugung von typischen Geräuschen und Lauten vorgesehen ist, die bei realen Untersuchungen oder bei der natürlichen Geburt auftreten können.

6. Geburtensimulator nach Anspruch 5, **dadurch gekennzeichnet, daß** die Schallerzeuger im Mutterleibstorso **(1)** integriert sind.

7. Kindmodell für einen Geburtensimulator nach einem der vorhergehenden Ansprüche, **dadurch gekennzeichnet, daß** an dem Kindmodell **(2)** im Halsbereich und/oder im Bereich des Schädeldaches, das aus verformbaren Segmenten besteht, Weg- und/oder Kraft- und/oder Drucksensoren **(13, 14)** angeordnet sind, die signaltechnisch mit der Steuervorrichtung verbunden sind.

## Claims

1. A birth simulator with the following features:
- a womb torso **(1)** of flexible material,
- a child model **(2)** of flexible material, which is arranged in the womb torso **(1)**, wherein the natural shape and size ratios and haptics are preferably maintained, the child model **(2)** is connected to
- a controllable drive **(5)** via a coupling device **(7)** in order to move the child model **(2)** in the womb torso **(1)** or expel it from the womb torso **(1)** through the birth canal, and
- a programmable control device for controlling the drive **(5)**, which comprises a computer, is provided,
**characterized in that**
- a sensor arrangement is provided for detecting forces and movements exerted onto the womb torso **(1)** or the child model **(2)** by an examining individual using the hands or medical instruments, wherein the sensors of the sensor arrangement are fixed on the coupling device **(7)** and/or the driving elements **(5)** and
- the control device comprises
- an admittance control or
- an impedance control
for controlling the drive elements **(5)**, wherein the control device is configured so that the measuring signals supplied by the sensor arrangement are sent to the computer, in which
- a simulation program is stored, which, when an exercising individual exerts external forces onto the child model **(2)**, co-operates with the admittance control or the impedance control of the control device to cause the driving elements **(5)** to move the child model **(2)** so that its movements correspond to the natural movement behavior of a child in its mother's womb during a certain examination or a certain phase of birth.

2. A birth simulator according to claim 1, **characterized in that** several coupling devices **(7)** and several controllable drives **(5)** for moving the child model **(2)** are provided.

3. A birth simulator according to any of the preceding claims, **characterized in that** an optic display device is provided, which is connected to the control device via a signal path and indicates the movements of child in real time.

4. A birth simulator according to claim 3, **characterized in that** the optic display device indicates directions and additional information.

5. A birth simulator according to any of the preceding claims, **characterized in that** an acoustic generator is provided, which is connected to the control device via a signal path and generates typical noises and sounds, which can occur during real examinations or during natural birth.

6. A birth simulator according to claim 5, **characterized in that** the acoustic generators are integrated in the womb torso **(1)**.

7. A child model for a birth simulator according to any of the preceding claims, **characterized in that** distance sensors and/or force sensors and/or pressure sensors **(13, 14)** are arranged in the neck region and/or in the skullcap region of the child model **(2)**, which consists of formable segments, and are connected to the control device via a signal path.

## Revendications

1. Simulateur d'accouchement avec les caractéristiques suivantes :
- un corps d'utérus (1) en matériau flexible,
- un foetus artificiel (2) en matériau flexible, qui est agencé dans le corps d'utérus, les rapports de forme et de dimension naturelles et l'haptique étant respectés, le foetus artificiel (2) étant relié à
- un dispositif d'accouplement (7) avec un système d'entraînement (5) apte à être commandé, pour faire bouger le foetus artificiel (2) dans le corps d'utérus (1) ou pour le pousser à travers le canal d'accouchement hors du corps d'utérus (1) et
- un dispositif de commande programmable, comportant un ordinateur, étant prévu pour commander le système d'entraînement (5),
**caractérisé en ce que**
- il est prévu un système de capteurs destinés à détecter les forces et les mouvements qui sont appliqués par une personne effectuant un examen, au moyen des mains ou d'instruments médicaux, sur le corps d'utérus (1) ou sur le foetus artificiel (2), les capteurs du système de capteurs étant agencés sur le dispositif d'accouplement (7) et/ou sur les éléments d'entraînement (5), et
- le dispositif de commande comporte un
-- réglage d'admittance ou un
-- réglage d'impédance
pour activer les éléments d'entraînement (5), le dispositif de commande étant réalisé de telle sorte que les signaux de mesure délivrés par le système de capteurs sont acheminés vers l'ordinateur, dans lequel est stocké
- un programme de simulation qui, en coopération avec le réglage d'admittance ou le réglage d'impédance du dispositif de commande, fait en sorte que le foetus artificiel (2) bouge, lors de l'application d'une force extérieure au moyen des éléments d'entraînement (8), de telle sorte que le foetus artificiel (2) effectue des mouvements qui correspondent au comportement de mouvement d'un foetus dans le ventre de la mère, lors de l'examen concerné ou lors de l'accouchement concerné.

2. Simulateur d'accouchement selon la revendication 1, **caractérisé en ce que** plusieurs dispositifs d'accouplement (7) et plusieurs systèmes d'entraînement (5) aptes à être commandés sont prévus pour faire bouger le foetus artificiel (2).

3. Simulateur d'accouchement selon l'une quelconque des revendications précédentes, **caractérisé en ce qu'**il est prévu un dispositif d'affichage optique, qui communique par la technique des signaux avec le dispositif de commande et qui représente les mouvements du foetus en temps réel.

4. Simulateur d'accouchement selon la revendication 3, **caractérisé en ce que** le dispositif d'affichage optique affiche des indications et des informations supplémentaires.

5. Simulateur d'accouchement selon l'une quelconque des revendications précédentes, **caractérisé en ce qu'**il est prévu un générateur de sons, communicant par la technique des signaux avec le dispositif de commande et destiné à générer des bruits et sons typiques, qui peuvent se produire lors d'examens réels ou lors d'un accouchement naturel.

6. Simulateur d'accouchement selon la revendication 5, **caractérisé en ce que** le générateur de sons est intégré dans le corps d'utérus (1).

7. Foetus artificiel pour un simulateur d'accouchement selon l'une quelconque des revendications précédentes, **caractérisé en ce que** des capteurs de déplacement et/ou de force et/ou de pression (13, 14), qui communiquent par la technique des signaux avec le dispositif de commande, sont agencés sur le foetus artificiel (2) dans la zone du cou et/ou dans la zone du sommet du crâne, qui est formé par des segments déformables.
